# FASCICULE DE BREVET EUROPEEN

(11) **EP 0 712 330 B1**
(45) Date de publication et mention de la délivrance du brevet: **01.10.1997**
(21) Numéro de dépôt: 94924335.6
(22) Date de dépôt: 05.08.1994
(51) Int. Cl.: B01F 17/00, B01F 17/56, C11D 1/94, C11D 1/66, A61K 7/00, A61K 47/00

(54) **COMPOSITIONS AQUEUSES CONCENTREES D'ALKYLPOLYGLYCOSIDES ET LEURS UTILISATIONS**
WÄSSRIGE ALKYLPOLYGLYCOSIDKONZENTRATE UND IHRE VERWENDUNG
CONCENTRATED AQUEOUS COMPOSITIONS OF ALKYLPOLYGLYCOSIDES, AND APPLICATIONS THEREOF

(30) Priorité: 06.08.1993 FR 9309735
(43) Date de publication de la demande: 22.05.1996
(73) Titulaire: S.E.P.P.I.C., SOCIETE D'EXPLOITATION DE PRODUITS POUR LES INDUSTRIES CHIMIQUES, F-75007 Paris (FR)
(72) Inventeur: LECOCU-MICHEL, Nelly, F-94700 Maisons-Alfort (FR); AMALRIC, Chantal, F-91700 Blan (FR)
(74) Mandataire: Hubert, Philippe
(86) Numéro de dépôt international: FR9400983
(87) Numéro de publication internationale: WO9504592

(56) Documents cités:
- EP-A- 0 379 398
- EP-A- 0 510 870
- WO-A-91/11506
- WO-A-92/06161
- WO-A-93/07160
- WO-A-93/07249
- JP-A- 3 269 097
- US-A- 4 668 422
- DATABASE WPI, Derwent Publications Ltd., London (GB); AN 92-020327

## Description

La présente invention concerne de nouvelles compositions aqueuses concentrées d'alkylpolyglycosides, fluides et homogènes, même à basse température, ainsi que des utilisations de ces compositions, notamment dans les domaines des produits d'hygiène, de la cosmétique et de la détergence ou dans les procédés de forage.

Les alkylpolyglycosides sont des tensio-actifs non ioniques bien connus. Ils peuvent notamment être préparés selon le procédé décrit dans las demandes de brevet EP-A-77.167 et EP-A-92.876. Ils peuvent être utilisés seuls ou, plus généralement, associés à d'autres tensio-actifs, dans de nombreuses applications. A cet égard, on peut faire référence aux demandes de brevet EP-A-70.074, WO-A-91/11506, WO-A-93/07160 EP-A-510 870, WO-A-92/06161, JP-A-03269097 et au brevet US-A-4.668.422 qui décrivent des compositions et formulations associant des alkylpolyglycosides et des tensio-actifs anioniques amphotères et/ou non ioniques, utiles notamment en détergence ou en cosmétique.

Les alkylpolyglycosides sont généralement commercialisés sous forme de compositions aqueuses concentrées, pouvant comporter plus de 12 % en poids, le plus souvent plus de 20% en poids, d'au moins un alkylpolyglycoside. Ces compositions concentrées sont utilisées pour la fabrication de différents types de formulations ou compositions dans des domaines d'application divers, tels ceux mentionnés ci-dessus. Il est notable que ces compositions concentrées ne sont pas elles-mêmes utilisées en tant que détergents ou cosmétiques.

Ces compositions aqueuses concentrées comportant essentiellement de l'eau et des alkylpolyglycosides peuvent être transportées ou stockées dans des récipients tel des containers ou des citernes de tailles variables. Or la demanderesse a pu constater que dans ces conditions, en fonction de la température ambiante, généralement à des températures comprises entre 0 et 20°C, des cristaux pouvaient se former au sein de ladite composition aqueuse concentrée ; cela parfois en moins d'une journée.

Les cristaux ont pour inconvénient de rendre trouble et hétérogène aussi bien la composition concentrée elle-même, que les compositions et formulations dans lesquelles elle est utilisée.

De plus, si l'on n'y prend garde, la cristallisation peut rapidement s'étendre à toute la composition concentrée qui prend alors en masse. Cette prise en masse peut même se produire préalablement à toute apparition de cristaux.

Dès lors, on recommande aux utilisateurs de ces compositions aqueuses concentrées d'alkylpolyglycosides de les surveiller régulièrement et, dès l'apparition de cristaux, de les chauffer à des températures de l'ordre de 40°C. Ainsi, ladite composition peut redevenir fluide et homogène.

Mais on comprend alors que ces phénomènes de cristallisation constituent un lourd handicap, notamment d'un point de vue économique, pour une bonne utilisation desdites compositions aqueuses concentrées.

Un premier objet de la présente invention consiste alors en des compositions aqueuses concentrées d'alkylpolyglycosides pouvant être utilisées pour préparer, notamment, des compositions détergentes ou cosmétiques, dans lesquelles la formation de cristaux et la prise en masse à basse température sont empêchées, ou tout au moins considérablement réduites et ralenties ; ces compositions restant fluides et homogènes.

Un autre objet de l'invention consiste en l'utilisation de ces compositions aqueuses concentrées d'alkylpolyglycosides pour la préparation de compositions ou de formulations, notamment dans les domaines des produits d'hygiène, de la cosmétique et de la détergence ou dans les procédés de forage.

L'invention consiste en une composition aqueuse concentrée d'alkylpolyglycosides, utile notamment pour la préparation d'une composition détergente hygiénique, cosmétique, pharmaceutique ou d'une composition pour forage, caractérisée en ce qu'elle comprend :
(i) plus de 12% en poids d'un ou plusieurs alkylpolyglycosides, la chaîne grasse de chacun de ces alkylpolyglycosides comportant au moins 10 atomes de carbone ; et
(ii) un tensio-actif amphotère permettant de prévenir ou de ralentir la prise en masse ou la formation de cristaux dans la composition aqueuse concentrée,
   ladite composition aqueuse concentrée étant exempte de tensio-actifs anioniques, non ioniques autres que des alkylpolyglycosides et d'alkanolamides d'acides gras.

La demanderesse a en effet pu constater que la présence d'un tensio-actif amphotère dans une composition aqueuse concentrée d'alkylpolyglycosides permettait d'empêcher, ou tout au moins de réduire et ralentir la formation de cristaux, de sorte à maintenir cette composition homogène et fluide.

Par ailleurs, et de manière surprenante, il a pu être constaté que la présence d'un tensio-actif amphotère ne modifie pas, ou très peu le pouvoir mouillant et le pouvoir moussant de la composition aqueuse concentrée d'alkylpolyglycosides.

Autrement dit une composition aqueuse concentrée selon l'invention peut être utilisée pour préparer une composition détergente ou cosmétique, par exemple, dans les mêmes conditions que la composition aqueuse classique, ne comportant substantiellement que des alkylpolyglycosides et de l'eau.

Cela présente un grand intérèt pour les utilisateurs qui peuvent préparer leur composition finale sans substantiellement en modifier la formulation.

Les alkylpolyglycosides entrant dans le cadre de la présente invention peuvent être ceux représentés par la formule (I) suivante

R - O - [(CH₂)ₓO]_{y}(G)ₙ (I)

dans laquelle R est un radical aliphatique, linéaire ou ramifié, saturé ou insaturé, comportant de 10 à 24 atomes de carbone, G est un reste d'un saccharide, x est un entier compris entre 2 et 4, y est un nombre compris entre 0 et 10, et n est un nombre compris entre 1 et 10.

Dans la formule (I), la chaîne grasse R représente de préférence un radical alkyle et comporte plus particulièrement de 10 à 18, plus préférentiellement de 10 à 16 atomes de carbone.

G peut être le reste d'un saccharide comportant 5 ou 6 atomes de carbone, comme par exemple le glucose, le mannose, le galactose, l'altrose, l'idose, l'arabinose, le xylose, le ribose, le gulose, le lyxose ou le fructose ; le glucose étant un saccharide préféré.

La valeur de n, qui représente le degré moyen de polymérisation du saccharide, peut plus particulièrement être comprise entre 1,05 et 5, de préférence entre 1,05 et 2,5. Des alkylpolyglycosides plus particulièrement concernés par l'invention sont ceux pour lesquels y est égal à 0.

Une composition aqueuse concentrée selon l'invention peut comporter de 15 à 70 % en poids, de préférence de 20 à 40% en poids d'au moins un alkylpolyglycoside, tel que défini ci-dessus.

Le plus souvent, une telle composition comporte de un à quatre desdits alkylpolyglycosides, qui diffèrent les uns des autres par la longueur de leur chaîne grasse.

Outre des alkylpolyglycosides présentant une chaîne grasse comportant au moins 10 atomes de carbone, une composition aqueuses concentrée selon l'invention peut également comporter une plus faible concentration en au moins un alkylpolyglycoside dont la chaîne grasse comporte moins de 10 atomes de carbone.

De tels alkylpolyglycosides peuvent plus particulièrement être ceux représentés par la formule (II) suivante :

R' - O - [(CH₂)ₓO]_{y}(G)ₙ (II)

dans laquelle G, x, y et n ont les significations indiquées ci-dessus et R' représente une chaîne aliphatique, linéaire ou ramifiée, saturée ou insaturée, comportant de 4 à 9 atomes de carbone.

De préférence R' est un radical alkyle comportant 4, 6 ou 8 atomes de carbone.

La teneur en poids de ces alkylpolyglycosides a chaîne grasse R' comportant moins de 10 atomes de carbone dans une composition aqueuses concentrée selon l'invention, peut être inférieure à 25 %, de préférence comprise entre 2 et 12 % en poids par rapport au poids total de ladite composition.

La composition selon l'invention peut comporter de 5 à 50% en poids, de préférence de 7 à 15 % en poids d'un tensio-actif amphotère. Les tensio-actifs amphotères pouvant être mis en oeuvre dans le cadre de la présente invention sont notamment décrits dans Galénica, Technique et Documentation Lavoisier, Volume V, Paris, 1983. De tels tensio-actifs amphotères peuvent être choisis parmi les N-alkylbétaïnes, les C-alkylbétaïnes, les N-alkylaminobétaïnes, les alkylamidobétaines, les alkylphosphoamidobétaïnes ou les alkylphosphobétaïnes, la sultaïne et ses dérivés, tels les alkylamidosulfobétaïnes, les dérivés de l'imidazoline, les N-alkylglycines, les N-alkyl-β-alanine, les alkylpolyaminecarboxylates et les N-alkylaminobutyrates.

A titre de tensio-actifs amphotères, on peut plus particulièrement citer la cocoamidobétaïne, la cocobétaïne, la cocoamidosultaïne, la cocoamidopropylbétaïne, la stéarylbétaïne, la stéarylamphocarboxylique glycinate, la cocoamidopropylhydroxysulfobétaïne, la cocoamphocarboxy-glycinate, la cocoimidazoline carboxylate ou la cocoamidopropyl diméthylamino hydroxy propyle d'hydrolysat de Collagène (selon nomenclature CTFA-Cosmetic Toiletries Fragrance Association).

Le rapport pondéral entre l'ensemble des alkylpolyglycosides et le tensio-actif amphotère constitutifs de la composition aqueuse concentrée selon l'invention, peut être supérieur à 0,5 et de préférence, il est compris entre 1,5 et 8.

Une composition aqueuse concentrée selon l'invention peut demeurer à une température inférieure à 20°C, plus généralement à une température comprise entre O°C et 20°C, cela pendant plus d'une semaine, le plus souvent pendant trois à 6 mois, sans que des cristaux ne se forment.

Selon la nature et les concentrations des alkylpolyglycosides et des tensio-actifs amphotères mis en oeuvre, les compositions aqueuses concentrées selon l'invention peuvent se voir conférer une viscosité accrue par rapport à une même composition ne comportant pas de tensio-actifs amphotères. Cependant, même dans ces conditions, les compositions selon l'invention restent fluides et donc pompables.

Avantageusement, une composition aqueuses concentrée selon l'invention présente une viscosité, mesurée à 20°C, inférieure à 8000 mPa.s, de préférence comprise entre 50 et 5000 mPa.s (Brookfield LVT).

Une composition aqueuse concentrée selon l'invention peut être préparée par simple mélange d'une composition aqueuse concentrée d'alkylpolyglycosides avec un tensio-actif amphotère.

Un autre objet de l'invention consiste en un procédé pour prévenir ou ralentir la prise en masse ou la formation de cristaux dans une composition aqueuse concentrée d'alkylpolyglycosides présentant notamment des chaîne grasses comportant plus de 10 atomes de carbone, tels ceux définis ci-dessus, selon lequel procédé, on mélange à ladite composition un tensio-actif amphotère.

Selon encore un autre objet, l'invention consiste en l'utilisation d'un tensio-actif amphotère, tel que défini plus haut, pour prévenir ou ralentir la prise en masse ou la formation de cristaux dans une composition aqueuse concentrée d'alkylpolyglycosides comportant plus de 12% en poids d'un ou plusieurs alkylpolyglycosides comportant au moins 10 atomes de carbones. Ces alkylpolyglycosides peuvent être tels que définis ci-dessus.

Selon un autre aspect, l'invention consiste en l'utilisation de compositions aqueuses concentrées d'alkylpolyglycosides décrites ci-dessus, pour la préparation des compositions ou de formulations dans divers domaines d'application, notamment en cosmétique, pharmacie, détergence et dans le domaine des produits d'hygiène. Les compositions selon l'invention peuvent encore être utilisées pour préparer des compositions pour forage.

Les compositions selon l'invention, peuvent ainsi être associées à des composés actifs ou des excipients conventionnels dans ces domaines d'applications.

Selon le domaine d'application, lesdites compositions ou formulations peuvent comporter de 0,1 à 10 % en poids en au moins un alkylpolyglycoside tel que défini plus haut.

Les exemples qui suivent ont pour but d'illustrer la présente invention.

### Exemple 1

On a préparé par simple mélange de ces constituants, une composition aqueuse concentrée comportant (% en poids):

| | |
|---|---|
| Oramix NS 10 ⁽¹⁾ | 30,4 % en matière active |
| Amonyl 380 BA ⁽²⁾ | 8,9 % en matière active |
| eau | qs |

La viscosité de cette composition, mesurée à 20°C, était de 2600 mPa.s. (Brookfield LVT, mobile 3, vitesse 6 tours/min.).
⁽¹⁾ mélange d'alkylpolyglycosides à chaîne grasse C₁₀ (85%), C₁₂ (7,5%) et C₁₄ (7,5 %), commercialisé par la Société Seppic, France.
⁽²⁾ tensio-actif amphotère du type cocamidopropylbétaïne commercialisé par la Société Seppic, France.

On a maintenu cette composition à 4°C pendant plus de 3 mois. Aucun phénomène de cristallisation n'a pu être constaté ; la composition est restée fluide et homogène.

A titre comparatif, une composition similaire mais ne comprenant pas de tensio-actif amphotère, cristallise en moins de 7 jours. Cette dernière composition présentait une viscosité, mesurée dans les conditions indiqués ci-dessus, de 200 mPa.s.

### Exemple 2

On prépare une composition aqueuse concentrée dans les conditions de l'exemple 1, comportant (% en poids) :

| | |
|---|---|
| Oramix NS 10 | 20 % en matière active |
| Amonyl 265 BA ⁽³⁾ | 20 % en matière active |
| eau | qs |

| | |
|---|---|
| ⁽³⁾ tensio-actif amphotère du type cocobétaïne commercialisé par la Société Seppic, France; | |

La viscosité de cette composition mesurée dans les conditions de l'exemple 1, est de 1.100 mPa.s.

Cette composition a été maintenue plus de 3 mois à 4°C, Aucune cristallisation n'a été constatée, cette composition est restée fluide et homogène.

A titre comparatif, on a préparé une composition similaire ne comportant pas de tensio-actif amphotère ; cette composition présentant une viscosité de 100 mPa.s.

Au bout de quatre jours, des cristaux en nombre abondant s'étaient formés dans cette dernière composition.

### Exemple 3

On prépare une composition aqueuse concentrée d'alkylpolyglycosides comportant (% en poids) :

| | |
|---|---|
| - alkylpolyglycoside en C₁₂ | 12,25 % en matière active |
| - alkylpolyglycoside en C₁₄ | 4,30 % en matière active |
| - alkylpolyglycoside en C₁₆ | 0,87 % en matière active |
| - Amonyl 265 BA | 19,5 % en matière active |
| - eau | qs |

Le degré de polymérisation moyen de chacun des alkylpolyglycosides de la composition est de 1,45.

La viscosité de cette composition, mesurée dans les conditions de l'exemple 1, est de 2.000 mPa.s.

Après plus de 3 mois à 4°C, aucun phénomène de cristallisation n'a été détectée ; cette composition est restée fluide et homogène.

A titre comparatif, on a reproduit l'essai ci-dessus avec une composition similaire, mais ne comportant pas le tensio-actif amphotère. La viscosité de cette composition était également de 2.000 mPa.s. Cette composition a cristallisé en moins de 7 jours.

### Exemple 4

En vue de démontrer qu'une composition aqueuse concentrée d'alkylglycosides selon l'invention présentait des propriétés moussantes et mouillantes similaires, voire identiques, à celles d'une composition aqueuse concentrée selon l'art antérieur, on a comparé le mouillage et le volume de mousse d'une composition Oramix NS 10 avec celles des compositions selon l'invention des exemples 1 et 2.

Les résultats obtenus figurent dans le tableau ci-dessus:

| | Oramix NS 10 | Composition de l'exemple 1 | Composition de l'exemple 2 |
|---|---|---|---|
| Mouillage(s) | 61 | 72 | 59 |
| Mousse (ml) eau dure + salissure, 1% extrait sec | 499 | 499 | 483 |

Le pouvoir mouillant a été mesuré selon la méthode AFNOR NFT 73-406.

Le volume de mousse a été mesuré selon la méthode ISO 696-1975.

Ces résultats montrent clairement que les propriétés moussantes et mouillantes d'une composition aqueuse concentrée selon l'invention ne sont pas modifiés par l'ajout d'un tensio-actif amphotère. Une telle composition selon l'invention peut donc être mise en oeuvre pour préparer des compositions, par exemple détergentes ou cosmétiques, sans que l'utilisateur n'ait à modifier sa formulation.

## Revendications

1. Composition aqueuse concentrée d'alkylpolyglycosides, utile notamment pour la préparation d'une composition détergente hygiénique, cosmétique, pharmaceutique ou d'une composition pour forage, caractérisée en ce qu'elle comprend :
(i) plus de 12% en poids d'un ou plusieurs alkylpolyglycosides, la chaîne grasse de chacun de ces alkylpolyglycosides comportant au moins 10 atomes de carbone ; et
(ii) un tensio-actif amphotère permettant de prévenir ou de ralentir la prise en masse ou la formation de cristaux dans la composition aqueuse concentrée,
ladite composition aqueuse concentrée étant exempte de tensio-actifs anioniques, de tensio-actifs
non ioniques autres que des alkylpolyglycosides et d'alkanolamides d'acides gras.

2. Composition selon la revendication 1, caractérisée en ce que ledit alkylpolyglycoside est un composé de formule (I) :
R - O - [(CH₂)ₓO]_{y}(G)ₙ (I)
dans laquelle R est un radical aliphatique, linéaire ou ramifié, saturé ou insaturé, comportant de 10 à 24 atomes de carbone, G est le reste d'un saccharide, x est un entier compris entre 2 et 4, y est un nombre compris entre 0 et 10, et n est un nombre compris entre 1 et 10,

3. Composition selon l'une des revendication 1 et 2, caractérisée en ce que G est le reste d'un glucose.

4. Composition selon l'une des revendications 1 à 3, caractérisée en ce que n est compris entre 1,05 et 5, de préférence entre 1,05 et 2,5, et y est égal à 0.

5. Composition selon l'une des revendications 1 à 4, caractérisée en ce que R est un radical alkyle comportant de 10 à 18 atomes de carbone, de préférence de 10 à 16 atomes de carbone.

6. Composition selon l'une des revendications 1 à 5 caractérisée en ce qu'elle comprend de 15 à 70 % en poids, de préférence de 20 à 40 % en au moins un alkylpolyglycoside dont la chaîne grasse comporte au moins 10 atomes de carbone.

7. Composition selon l'une des revendications 1 à 6, caractérisée en ce que ledit tensio-actif amphotère est choisi parmi les alkylbétaïnes, les alkylaminobétaïnes, les alkylamidobétaïnes, les alkylphosphoamidobétaïnes, les alkylphosphobétaïnes, la sultaïne et ses dérivés, tels les alkylamidosulfobétaïnes, les dérivés de l'imidazoline, les N-alkylglycine, les N-alkyl-β-alanine, les alkylpolyaminecarboxylates et les N-alkylaminobutyrates.

8. Composition selon l'une des revendications 1 à 7, caractérisée en ce qu'elle comprend de 5 à 50 % en poids, de préférence de 7 à 15 % en poids d'un tensio-actif amphotère.

9. Composition selon l'une des revendications 1 à 8, caractérisée en ce que le rapport pondéral entre l'alkylpolyglycoside et le tensio-actif amphotère est supérieur à 0,5, de préférence compris entre 1,5 et 8.

10. Composition selon l'une des revendications 1 à 9 caractérisée en ce qu'elle comporte en outre un alkylpolyglycoside dont la chaîne grasse comporte moins de 10 atomes de carbone, en une teneur inférieure à 25 %, de préférence comprise entre 2 et 12 %, en poids par rapport au poids total de ladite composition.

11. Procédé pour prévenir ou ralentir la prise en masse ou la formation de cristaux dans une composition aqueuse concentrée d'alkylpolyglycosides comprenant plus de 12% en poids d'un ou plusieurs alkylpolyglycosides, la chaîne grasse de chacun de ces alkylpolyglycosides comportant au moins 10 atomes de carbone caractérisé en ce qu'on mélange à ladite composition un tensio-actif amphotère.

12. Procédé selon la revendication 11, caractérisé en ce que la teneur en tensio-actif amphotère dans ladite composition est comprise entre 5 et 50 % en poids, de préférence entre 7 et 15 % en poids.

13. Utilisation d'un tensio-actif amphotère pour prévenir ou ralentir la prise en masse ou la formation de cristaux dans une composition aqueuse concentrée d'alkylpolyglycosides comprenant plus de 12% en poids d'un ou plusieurs alkylpolyglycosides, la chaîne grasse de chacun de ces alkylpolyglycosides comportant au moins 10 atomes de carbone.

14. Utilisation d'une composition selon l'une des revendications 1 à 10 pour la préparation d'une composition ou d'une formulation cosmétique, pharmaceutique, détergente ou destinée à l'hygiène, ou d'une composition pour forage.

## Claims

1. Concentrated aqueous composition of alkylpolyglycosides, of use in particular for the preparation of a hygienic, cosmetic or pharmaceutical composition or a composition for drilling, characterized in that it comprises :
(i) more than 12 % by weight of one or more alkylpolyglycosides, the fatty chain of each of these alkylpolyglycosides having at least 10 carbon atoms; and
(ii) an amphoteric surfactant preventing or retarding the setting of, or the formation of crystals in, the concentrated aqueous composition;
the said concentrated aqueous composition being free from anionic surfactants and non-ionic surfactants other than alkylpolyglycosides and fatty acid alkanolamides.

2. Composition according to claim 1, characterized in that the said alkylpolyglycoside is a compound with the formula (I) :
R - O - [(CH₂)ₓO]_{y}(G)ₙ (I)
in which R is a saturated or unsaturated, linear or branched aliphatic radical having 10 to 24 carbon atoms, G is a saccharide residue, x is an integer between 2 and 4, y is a number between 0 and 10 and n is a number between 1 and 10.

3. Composition according to either of claims 1 or 2, characterized in that G is a glucose residue.

4. Composition according to one of claims 1 to 3, characterized in that n is between 1.05 and 5, preferably between 1.05 and 2.5, and y is equal to 0.

5. Composition according to one of claims 1 to 4, characterized in that R is an alkyl radical having 10 to 18 carbon atoms, preferably 10 to 16 carbon atoms.

6. Composition according to one of claims 1 to 5, characterized in that it comprises 15 to 70 %, preferably 20 to 40 %, by weight of at least one alkylpolyglycoside of which the fatty chain has at least 10 carbon atoms.

7. Composition according to one of claims 1 to 6, characterized in that the said amphoteric surfactant is selected from alkylbetaines, alkylaminobetaines, alkylamidobetaines, alkylphosphoamidobetaines, alkylphosphobetaines, sultaine and its derivatives such as alkylamidosulphobetaines, imidazoline derivatives, N-alkylglycines, N-alkyl-β-alanines, alkylpolyaminecarboxylates and N-alkylaminobutyrates.

8. Composition according to one of claims 1 to 7, characterized in that it comprises 5 to 50 % by weight, preferably 7 to 15 % by weight, of an amphoteric surfactant.

9. Composition according to one of claims 1 to 8, characterized in that the weight ratio between the alkylpolyglycoside and the amphoteric surfactant is greater than 0.5, preferably between 1.5 and 8.

10. Composition according to one of claims 1 to 9, characterized in that it additionally comprises an alkylpolyglycoside of which the fatty chain has less than 10 carbon atoms, in a concentration of less than 25 %, preferably between 2 and 12 %, by weight based on the total weight of the said composition.

11. Process for preventing or retarding the setting of, or the formation of crystals in, a concentrated aqueous composition of alkylpolyglycosides comprising more than 12 % by weight of one or more alkylpolyglycosides, the fatty chain of each of these alkylpolyglycosides having at least 10 carbon atoms, characterized in that an amphoteric surfactant is mixed with the said composition.

12. Process according to claim 11, characterized in that the concentration of amphoteric surfactant in the said composition is between 5 and 50 % by weight, preferably between 7 and 15 % by weight.

13. Use of an amphoteric surfactant for preventing or retarding the setting of, or the formation of crystals in, a concentrated aqueous composition of alkylpolyglycosides comprising more than 12 % by weight of one or more alkylpolyglycosides, the fatty chain of each of these alkylpolyglycosides having at least 10 carbon atoms.

14. Use of a composition according to one of claims 1 to 10 for the preparation of a hygienic, cosmetic or pharmaceutical composition or formulation or a composition for drilling.

## Patentansprüche

1. Konzentrierte, wässerige Zusammensetzung aus Alkylpolyglycosiden, verwendbar insbesondere zur Herstellung einer hygienischen, kosmetischen, pharmazeutischen Reinigungsverbindung oder einer Bohrhilfsflüssigkeit, gekennzeichnet durch
(i) mehr als 12 Gew.-% mindestens eines Alkylpolyglycosids, dessen aliphatische Kette mindestens zehn Kohlenstoffatome umfaßt; und
(ii) einen amphoteren, grenzflächenaktiven Stoff, der ermöglicht, das Wachstum oder die Bildung von Kristallen in der konzentrierten, wässerigen Zusammensetzung zu verhindern oder zu verlangsamen,
wobei die konzentrierte, wässerige Zusammensetzung frei von anionischen, grenzflächenaktiven Stoffen, von nicht ionischen, grenzflächenaktiven Stoffen, soweit es sich nicht um Alkylpolyglycoside handelt, und von Fettsäure-alkanolamiden ist.

2. Zusammensetzung nach Anspruch 1, dadurch gekennzeichnet, daß das Alkylpolyglycosid eine Verbindung mit der Formel (I)
R - O - [(CH₂)_{X}O] _{y} (G)ₙ (I)
ist, bei der R ein lineares oder verzweigtes, gesättigtes oder ungesättigtes, aliphatisches Radikal ist, das 10 bis 24 Kohlenstoffatome enthält, G der Rest eines Saccharids ist, X eine ganze Zahl zwischen 2 und 4 ist, Y eine Zahl zwischen 0 und 10 ist und N eine Zahl zwischen 1 und 10 ist.

3. Zusammensetzung nach einem der Ansprüche 1 und 2, dadurch gekennzeichnet, daß G der Rest einer Glucose ist.

4. Zusammensetzung nach einem der Ansprüche 1 bis 3, dadurch gekennzeichnet, daß n zwischen 1.05 und 5 und vorzugsweise zwischen 1.05 und 2.5 liegt und y gleich 0 ist.

5. Zusammensetzung nach einem der Ansprüche 1 bis 4, dadurch gekennzeichnet, daß R ein Alkylradikal ist, das 10 bis 18 Kohlenstoffatome und vorzugsweise 10 bis 16 Kohlenstoffatome enthält.

6. Zusammensetzung nach einem der Ansprüche 1 bis 5, dadurch gekennzeichnet, daß sie 15 bis 70 Gew.-%, vorzugsweise 20 bis 40 Gew.% mindestens eines Alkylpolyglycosids enthält, dessen aliphatische Kette mindestens zehn Kohlenstoffatome enthält.

7. Zusammensetzung nach einem der Ansprüche 1 bis 6, dadurch gekennzeichnet, daß der amphotere, grenzflächenaktive Stoff aus den Alkylbetainen, den Alkylaminobetainen, den Alkylamidobetainen, den Alkylphosphoamidobetainen, den Alkylphosobetainen, Sultain und dessen Derivate wie den Alkylamidosulfobetainen, den Derivaten von Imidazolin, den N-Alkylglyzinen, den N-Alkyl-β-Alaninen, den Alkylpolyamincarboxylaten und den N-Alkylaminobutyraten ausgewählt wird.

8. Zusammensetzung nach einem der Ansprüche 1 bis 7, dadurch gekennzeichnet, daß sie 5 bis 50 Gew.%, vorzugsweise 7 bis 15 Gew.-% eines amphoteren, grenzflächenaktiven Stoffes enthält.

9. Zusammensetzung nach einem der Ansprüche 1 bis 8, dadurch gekennzeichnet, daß das Gewichtsverhältnis zwischen dem Alkylpolyglykosid und dem amphoteren, grenzflächenaktiven Stoff größer als 0.5, vorzugsweise zwischen 1.5 und 8 ist.

10. Zusammensetzung nach einem der Ansprüche 1 bis 9, dadurch gekennzeichnet, daß sie ein Alkylpolyglycosid enthält, dessen aliphatische Kette mindestens zehn Kohlenstoffatome mit einem Mindestgehalt von 2 Gew.-% und vorzugsweise zwischen 2 und 12 Gew.-%, bezogen auf das Gesamtgewicht der Zusammensetzung, enthält.

11. Verfahren zum Verhindern oder Verlangsamen des Wachstums oder der Bildung von Kristallen in einer konzentrierten, wässerigen Zusammensetzung aus Alkylpolyglycosiden, die mehr als 12 Gew.-% mindestens eines Alkylpolyglycosids enthält, dessen aliphatische Kette mindestens 10 Kohlenstoffatome umfaßt, dadurch gekennzeichnet, daß der Zusammensetzung ein amphoterer, grenzflächenkativer Stoff beigemengt wird.

12. Verfahren nach Anspruch 11, dadurch gekennzeichnet, daß der Anteil des amphoteren, grenzflächenaktiven Stoffes in der Zusammensetzung zwischen 5 und 50 Gew.-% und vorzugsweise zwischen 7 und 15 Gew.-% liegt.

13. Verwendung eines amphoteren, grenzflächenaktiven Stoffs zum Verhindern oder Verlangsamen des Wachstums oder der Bildung von Kristallen in einer konzentrierten, wässerigen Zusammensetzung aus Alkylpolyglycosiden, die mehr als 12 Gew.-% mindestens eines Alkylpolyglycosids enthält, dessen aliphatische Kette jedes dieser Alkylpolyglycoside mindestens zehn Kohlenstoffatome umfaßt.

14. Verwendung einer Zusammensetzung nach einem der Ansprüche 1 bis 10 zur Herstellung einer kosmetischen, pharmazeutischen oder für die Hygiene bestimmten Verbindung oder Rezeptur oder einer Bohrhilfsflüssigkeit.
